# EUROPEAN PATENT APPLICATION

(11) **EP 4 342 479 A1**
(43) Date of publication of application: **27.03.2024**
(21) Application number: 22804992.0
(22) Date of filing: 18.05.2022
(51) Int. Cl.: A61K 35/28, A61P 17/00, A61P 29/00, C12N 5/0775, A61K 8/98, A61Q 19/00

(54) **COMPOSITION CONTAINING FUNCTION-REINFORCED STEM CELL FOR PREVENTION OR TREATMENT OF ATOPIC DERMATITIS**

(30) Priority: 18.05.2021 KR 20210064147
(71) Applicant: SCM Lifescience Co., Ltd., Incheon 21999 (KR)
(72) Inventor: SONG, Sun Uk, Incheon 22003 (KR); KIM, Si Na, Incheon 21997 (KR); CHOI, Byeol, Incheon 21970 (KR)
(74) Representative: Boult Wade Tennant LLP
(86) International application number: PCT/KR2022/007135
(87) International publication number: WO 2022/245138

(57) **Abstract**

The present invention relates to a composition containing primed, function-reinforced stem cells for preventing, alleviating or treating atopic dermatitis. The stem cells primed by treatment with TNF-α, IFN-γ, and IFN-α or with TNF-α, IFN-γ, IFN-α, and vitamins according to the present invention have reinforced anti-inflammatory and immunomodulatory ability and thus exhibit excellent prophylactic or therapeutic effects on atopic dermatitis symptoms, compared to stem cells that are not treated with the above priming factors. Therefore, the function-reinforced stem cells of the present invention can be widely utilized in various fields of prevention, alleviation, or treatment of atopic dermatitis.

## Description

### [Technical Field]

The present invention relates to a composition containing primed, function-reinforced stem cells for preventing, alleviating or treating atopic dermatitis.

### [Background Art]

Stem cells are undifferentiated cells, which can divide for a long time through self-renewal, and refer to cells capable of differentiating into various types of cells under a specific environment. The stem cells may be divided into embryonic stem cells and adult stem cells depending on an originating tissue, but treatment experiments using the embryonic stem cells have difficulties due to ethical aspects and tumorigenicity, whereas the adult stem cells have an advantage of being easily obtained from various tissues, and thus, studies are being actively conducted to apply the stem cells to the treatment of various diseases.

To date, many compound immunosuppressive agents or anti-inflammatory agents have been developed, and the clinically most commonly used immunosuppressive agents include cyclosporine (Neoral, Cipol A), azathioprine (Imuran), prednisolone (a kind of steroid), and the like. The immunosuppressive agent inhibits several processes, such as phagocytosis of antigens by macrophages, antigen recognition by lymphocytes, cell division, division of T cells and B cells, and antibody production, during a process from antigen stimulation to antibody production, thereby causing immunosuppression. Most of these drugs have antitumor activity at the same time, but the reason is that these drugs inhibit cell division through DNA disorders, inhibition of DNA synthesis, and the like. However, representative side effects according thereto include hypertension and nephrotoxicity (decreased kidney function), and since the incidence of these side effects is high, there are problems such as a need to observe the progress sufficiently when used. Other side effects rarely include trembles, seizures, hepatitis, bile retention, increased uric acid in the blood, decreased muscle strength, hypertrichosis, gingival hypertrophy, and the like. Among the commonly used inhibitors, azathioprine inhibits bone marrow functions such as decreased white blood cell count, anemia, and decreased platelets, and may have complications such as pancreatitis, hepatitis, and bile retention, and rarely hair loss and fever. Prednisolone, one of the steroid drugs, started to be first used among immunosuppressive agents, but is a drug to be careful about not only promoting arteriosclerosis, but also causing hypertension, gastric ulcers, diabetes, growth inhibition, osteoporosis, cataract, and glaucoma. Therefore, the need for safe immunosuppressive agents or anti-inflammatory agents has emerged.

In order to solve these problems, therapies using stem cells have recently been attempted for the treatment of inflammatory and immune diseases. In particular, mesenchymal stem cells (MSCs) are known to suppress inflammation, induce the generation of regulatory T cells (Treg), or induce the death of immune cells involved in apoptosis, and thus, the development of various therapeutic agents using the MSCs is being actively conducted.

However, in various clinical trials using stem cells, there have been reported problems that persistence of effects or *in vivo* survival rates as a result of long-term follow-up was extremely low, and thus, various attempts are being made to improve stem cell therapeutics. For example, in order to increase the therapeutic effect, there are attempts of a method of pre-processing various chemicals and peptides capable of inserting specific genes into stem cells or boosting stem cell functions, and methods of adding stimulation conditions such as hypoxia, temperature, and light during culture, or in order to increase the survival rate of stem cells, methods of administering the stem cells together with a support are being studied. Among various studies for reinforcing the stem cell functions, a function improvement method using genetic manipulation may be effective, but problems such as the safety of the introduced gene are pointed out.

Therefore, various studies have been attempted to reinforce the intrinsic functions of stem cells through treatment of various priming factors during culture rather than reinforcing the functions by introducing the genes into the stem cells themselves.

### [Disclosure]

### [Technical Problem]

The present inventors confirmed that TNF-α, IFN-γ and IFN-α; or TNF-α, IFN-γ, IFN-α, and vitamins were treated to significantly improve prophylactic or therapeutic effects of stem cells on atopic dermatitis while studying the stem cell functions to be further reinforced, and then completed the present invention.

Accordingly, an object of the present invention is to provide a composition containing stem cells primed by TNF-α, IFN-γ and IFN-α; or TNF-α, IFN-γ, IFN-α, and vitamins for preventing, alleviating or treating atopic dermatitis.

### [Technical Solution]

In order to achieve the object, an aspect of the present invention provides a pharmaceutical composition containing stem cells treated with TNF-α, IFN-γ, and IFN-α for preventing or treating atopic dermatitis.

Another aspect of the present invention provides a pharmaceutical composition containing stem cells treated with TNF-α, IFN-γ, IFN-α, and at least one vitamin selected from the group consisting of vitamin B2, vitamin B3, vitamin B5 and vitamin B6, for preventing or treating atopic dermatitis.

Yet another aspect of the present invention provides a stem cell therapeutic agent containing the composition for preventing or treating atopic dermatitis.

Yet another aspect of the present invention provides a cosmetic composition containing stem cells treated with TNF-α, IFN-γ, and IFN-α for preventing or alleviating atopic dermatitis.

Yet another aspect of provides a cosmetic composition containing stem cells treated with TNF-α, IFN-γ, IFN-α, and at least one vitamin selected from the group consisting of vitamin B2, vitamin B3, vitamin B5 and vitamin B6, for preventing or alleviating atopic dermatitis.

Yet another aspect of the present invention provides a method for preparing a composition for preventing or treating atopic dermatitis, including 1) culturing stem cells treated with TNF-α, IFN-γ, and IFN-α.

Yet another aspect of the present invention provides a method for preparing a composition for preventing or treating atopic dermatitis, including 1) culturing stem cells treated with TNF-α, IFN-γ, IFN-α, and at least one vitamin selected from the group consisting of vitamin B2, vitamin B3, vitamin B5 and vitamin B6.

Yet another aspect of the present invention provides a method for preventing or treating atopic dermatitis including treating stem cells primed by TNF-α, IFN-γ, and IFN-α; or TNF-α, IFN-γ, IFN-α, and vitamins to a subject in need thereof.

### [Advantageous Effects]

According to the present invention, the stem cells primed by treatment with TNF-α, IFN-γ, and IFN-α or with TNF-α, IFN-γ, IFN-α, and vitamins have reinforced anti-inflammatory and immunomodulatory ability and thus exhibit excellent prophylactic or therapeutic effects on atopic dermatitis symptoms, compared to stem cells that are not treated with the above priming factors. Therefore, the function-reinforced stem cells of the present invention can be widely utilized in various fields of prevention, alleviation, or treatment of atopic dermatitis.

### [Description of Drawings]

FIG. 1 is a diagram illustrating results of confirming increased expression of IDO and TSG6 according to a treatment concentration of TNF-α (***P < 0.001, ****P < 0.0001, compared to the control (0 ng/ml)).
FIG. 2 is a diagram illustrating results of confirming increased expression of IDO and TSG6 according to a treatment concentration of IFN-γ (*P < 0.05, ****P < 0.0001, compared to the control (0 ng/ml)).
FIG. 3 is a diagram illustrating results of confirming increased expression of IDO and TSG6 according to a treatment concentration of IFN-α (*p < 0.05, **p < 0.01, ***P < 0.001, ****P < 0.0001, compared to the control (0 ng/ml)).
FIG. 4 is a diagram illustrating results of confirming increased expression of IDO and TSG6 according to compositions shown in Table 1 (****P < 0.0001) (0: untreated control; 1: TNF-α (10 ng/ml); 2: IFN-γ (10 ng/ml); 3: IFN-α (20 ng/ml); 4: TNF-α (10 ng/ml) + IFN-γ (10 ng/ml); 5: TNF-α (10 ng/ml) + IFN-α (20 ng/ml); 6: IFN-γ (10 ng/ml) + IFN-α (20 ng/ml); 7: TNF-α (10 ng/ml) + IFN-γ (10 ng/ml) + IFN-α (20 ng/ml)).
FIG. 5 is a diagram illustrating results of confirming increased expression of IDO and TSG6 according to treatment of vitamins alone shown in Table 2 (0: untreated control; 1: Vitamin A (10 µg/ml); 2: Vitamin B1 (50 µg/ml); 3: Vitamin B2 (5 µg/ml); 4: Vitamin B3 (50 µg/ml); 5: Vitamin B5 (50 µg/ml); 6: Vitamin B6 (50 µg/ml); 7: Vitamin B12 (50 µg/ml); 8: Vitamin D2 (10 µg/ml); 9: Vitamin D3 (10 µg/ml)).

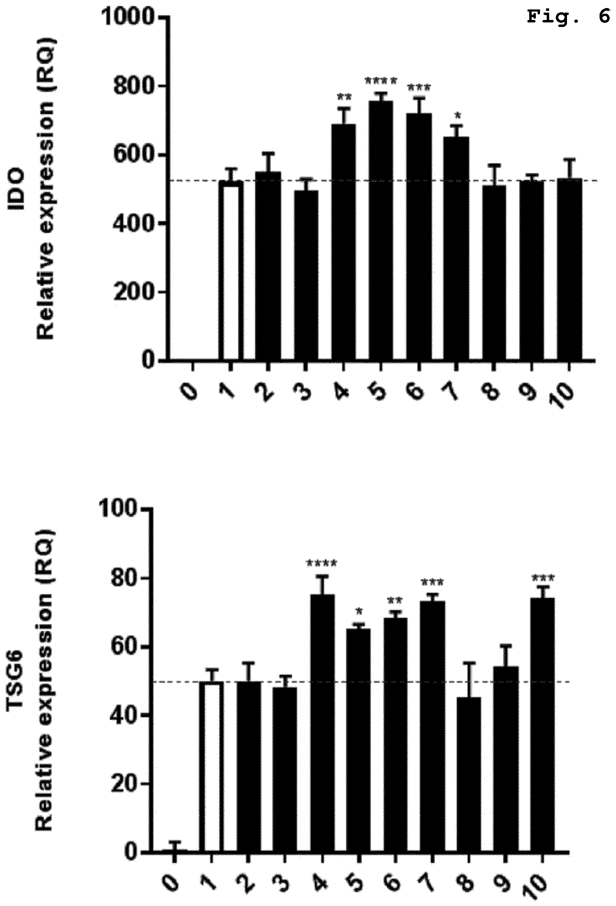

FIG. 6 is a diagram illustrating results of confirming increased expression of IDO and TSG6 according to the treatment with compositions shown in Table 3 in which vitamins are added to TNF-α, IFN-γ, and IFN-α, respectively (*P < 0.05, **P < 0.01, ***P < 0.001, ****P < 0.0001, compared to Group 1) (0: untreated control; 1: TNF-α + IFN-γ + IFN-α; 2: addition of vitamin A to Experimental Group 1; 3: addition of vitamin B1 to Experimental Group 1; 4: addition of vitamin B2 to Experimental Group 1; 5: addition of vitamin B3 to Experimental Group 1; 6: addition of vitamin B5 to Experimental Group 1; 7: addition of vitamin B6 to Experimental Group 1; 8: addition of vitamin B12 to Experimental Group 1; 9: addition of vitamin D2 to Experimental Group 1; 10: addition of vitamin D3 to Experimental Group 1).

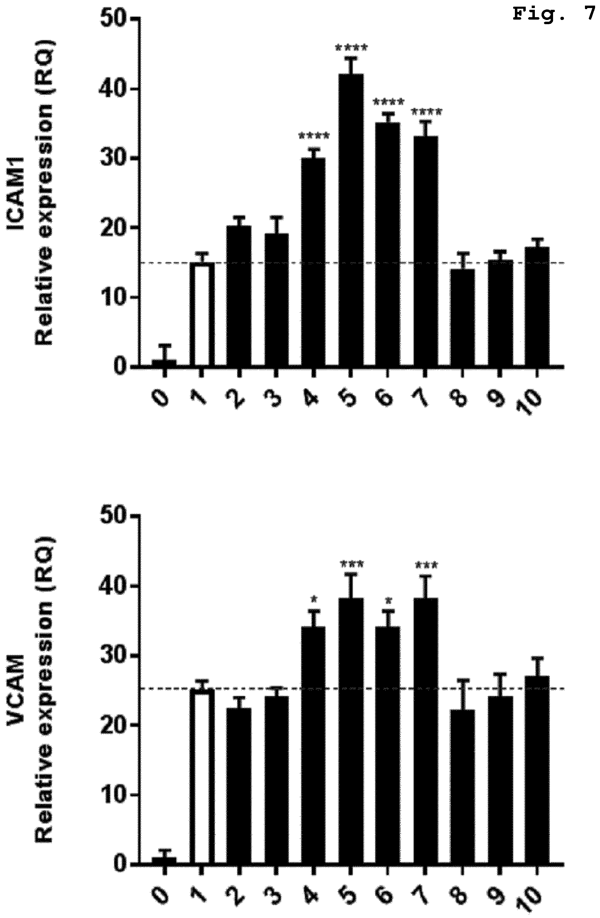

FIG. 7 is a diagram illustrating results of confirming increased expression of ICAM1 and VCAM according to the treatment with compositions shown in Table 3 in which vitamins are added to TNF-α, IFN-γ, and IFN-α, respectively. (*P < 0.05, **P < 0.01, ***P < 0.001, ****P < 0.0001, compared to Group 1) (0: untreated control; 1: TNF-α + IFN-γ + IFN-α; 2: addition of vitamin A to Experimental Group 1; 3: addition of vitamin B1 to Experimental Group 1; 4: addition of vitamin B2 to Experimental Group 1; 5: addition of vitamin B3 to Experimental Group 1; 6: addition of vitamin B5 to Experimental Group 1; 7: addition of vitamin B6 to Experimental Group 1; 8: addition of vitamin B12 to Experimental Group 1; 9: addition of vitamin D2 to Experimental Group 1; 10: addition of vitamin D3 to Experimental Group 1).
FIG. 8 is a diagram illustrating results of confirming inflammatory cytokine IFN-γ and anti-inflammatory cytokine IL-10 by co-culturing PHA-stimulated PBMCs with pcMSC2 or untreated cMSCs in order to confirm immunomodulatory ability of stem cells pcMSC2 treated with TNF-α, IFN-γ, IFN-α and vitamin B6 (*** P < 0.001, **** P < 0.0001, compared to cMSC, P1: negative control, not stimulated with PHA).
FIG. 9 is a diagram illustrating results of confirming changes in skin lesions according to administration of an untreated group (native), a control group (Cell Vehicle: PBS), cMSC, and pcMSC2 in an atopic dermatitis animal model.
FIG. 10 is a diagram illustrating results of confirming changes in skin lesions according to administration of an untreated group (native), a control group (Cell Vehicle: PBS), cMSC, and pcMSC2 in an atopic dermatitis animal model through Toluidine blue staining.
FIG. 11 is a diagram illustrating results of confirming changes in mast cell counts according to administration of an untreated group (native), a control group (Cell Vehicle: PBS), cMSC, and pcMSC2 in an atopic dermatitis animal model (* P < 0.05, compared to cMSC, ## P < 0.001, compared to vehicle).
FIG. 12 is a diagram illustrating results of confirming changes in total IgE production according to administration of an untreated group (native), a control group (Cell Vehicle: PBS), cMSC, and pcMSC2 in an atopic dermatitis animal model through ELISA assay (* P < 0.05, ** P < 0.01, **** P < 0.0001) (* P < 0.05, compared to cMSC, ## P < 0.001, compared to vehicle).
FIG. 13 is a diagram illustrating results of confirming changes in total IgG2a production according to administration of an untreated group (native), a control group (Cell Vehicle: PBS), cMSC, and pcMSC2 in an atopic dermatitis animal model through ELISA assay (* P < 0.05, *** P < 0.001).
FIG. 14 is a diagram illustrating results of confirming histamine changes according to administration of an untreated group (native), a control group (Cell Vehicle: PBS), cMSC, and pcMSC2 in an atopic dermatitis animal model through ELISA assay (*** P < 0.001, **** P < 0.0001).

### [Best mode of the Invention]

Hereinafter, the present invention will be described in detail.

The present invention provides a pharmaceutical composition containing stem cells treated with TNF-α, IFN-γ, and IFN-α; or TNF-α, IFN-γ, IFN-α, and at least one vitamin selected from the group consisting of vitamin B2, vitamin B3, vitamin B5 and vitamin B6, for preventing or treating atopic dermatitis.

Compared to stem cells not treated with TNF-α, IFN-γ and IFN-α; or TNF-α, IFN-γ, IFN-α and at least one vitamin selected from the group consisting of vitamins B2, B3, B5, and B6, the stem cells of the present invention have excellent anti-inflammatory and immunomodulatory ability, and thus may be effectively used for prevention or treatment of atopic dermatitis.

In the present invention, the stem cells treated with TNF-α, IFN-γ and IFN-α; or TNF-α, IFN-γ, IFN-α and at least one vitamin selected from the group consisting of vitamins B2, B3, B5, and B6 may be referred to as `primed stem cells' or 'function-reinforced stem cells' and the `primed stem cells' and the `function-reinforced stem cells' may be used interchangeably. The primed stem cells refer to stem cells that exhibit excellent atopic dermatitis therapeutic effects by significantly increasing the immunomodulatory and inflammatory regulation ability of stem cells by treatment with priming factors of the present invention.

In the present invention, the `priming factors' may mean a combination of TNF-α, IFN-γ, and IFN-α, or a combination of further containing at least one vitamin selected from the group consisting of vitamin B2, vitamin B3, vitamin B5, and vitamin B6 in the combination.

The combination of the priming factors may exhibit a synergistic effect compared to treating stem cells with these single ingredients, and effectively induce reinforcement of the function of desired stem cells even at a low treatment concentration.

In the present invention, 'function reinforcement' refers to the enhancement of the inherent properties and effects of stem cells by treatment with the priming factors, and particularly preferably, may mean improvement of an effect of preventing, alleviating or treating atopic dermatitis.

Specifically, the primed stem cells of the present invention may be stem cells treated with TNF-α, IFN-γ, and IFN-α. More specifically, the TNF-α, IFN-γ, and IFN-α may be treated in a ratio of 0.1 to 3 : 0.1 to 3 : 0.1 to 3 (w/v), preferably 1 : 1 : 0.1 to 3 (w/v), more preferably 1 : 1 : 1 to 3 (w/v), and much more preferably 1 : 1 : 1 to 2.5 (w/v). In one embodiment of the present invention, the stem cells were treated with TNF-α, IFN-γ, and IFN-α at a concentration combination of 10 ng/ml, 10 ng/ml, and 20 ng/ml, and cultured to confirm an atopic dermatitis therapeutic effect of the stem cells.

In addition, the stem cells of the present invention may be stem cells further treated with vitamins in addition to the combination of TNF-α, IFN-γ, and IFN-α, and specifically, stem cells further treated with at least one vitamin selected from the group consisting of vitamin B2, vitamin B3, vitamin B5, and vitamin B6. When the stem cells are primed by treatment with a combination of priming factors further containing vitamins, the reinforced function of the stem cells induced by treatment with TNF-α, IFN-γ and IFN-α may be more remarkably enhanced and the effect of preventing or treating atopic dermatitis may also be significantly improved. The TNF-α, IFN-γ, IFN-α and vitamins may be treated to the stem cells in a ratio of 0.1 to 3 : 0.1 to 3 : 0.1 to 3 : 100 to 10,000 (w/v), preferably 1 : 1 : 0.1 to 3 : 300 to 6,000 (w/v). More specifically, when vitamin B2 is added to TNF-α, IFN-γ, and IFN-α, the TNF-α, IFN-γ, IFN-α and vitamin may be treated in a ratio of 0.1 to 3 : 0.1 to 3 : 0.1 to 3 : 300 to 1,000 (w/v), more preferably 1 : 1 : 0.1 to 3 : 300 to 600 (w/v), much more preferably 1 : 1 : 0.1 to 3 : 400 to 550 (w/v), and even more preferably 1 : 1 : 2 : 500 (w/v). In addition, when vitamin B3, vitamin B5, or vitamin B6 is added to TNF-α, IFN-γ, and IFN-α, the TNF-α, IFN-γ, IFN-α and the vitamin may be treated in a ratio of 0.1 to 3 : 0.1 to 3 : 0.1 to 3 : 1,000 to 10,000 (w/v), more preferably 0.1 to 3 : 0.1 to 3 : 0.1 to 3 : 1,000 to 6,000 (w/v), much preferably 1 : 1 : 0.1 to 3 : 4,000 to 6,000 (w/v), even more preferably 1 : 1 : 0.1 to 3 : 4,000 to 5,500 (w/v), and even more preferably 1 : 1 : 2 : 5,000 (w/v).

In one embodiment of the present invention, as concentrations for combined treatment, 10 ng/ml of TNF-α, 10 ng/ml of IFN-γ, and 20 ng/ml of IFN-α were selected, and 5 µg/ml of vitamin B2, 50 µg/ml of vitamin B3, 50 µg/ml of vitamin B5 and 50 µg/ml of vitamin B6 were selected and treated to the stem cells to confirm the reinforcement of immunomodulatory and inflammatory regulation functions. As a result, in Experimental Groups added with vitamins, compared to treatment of TNF-α, IFN-γ, and IFN-α, it was confirmed that not only the expression of IDO and TSG6 was increased, but also the expression of intercellular adhesion molecule 1 (ICAM1) and vascular cell adhesion molecule (VCAM) as adhesion factors expressed on the surface of stem cells was significantly increased.

In the present invention, the `priming treatment' or `treatment of priming factors' may mean treating stem cells with a combination of TNF-α, IFN-γ, and IFN-α, or a combination of further containing at least one vitamin selected from the group consisting of vitamin B2, vitamin B3, vitamin B5, and vitamin B6 in the combination for 12 hours to 36 hours, preferably 20 hours to 25 hours, and then culturing the stem cells. For the culture, stem cell culture media widely known in the art may be used without limitation.

In the present invention, the primed stem cells functionally reinforced by treatment with TNF-α, IFN-γ, and IFN-α; or TNF-α, IFN-γ, IFN-α and the vitamins refer to cells having ability to differentiate into two or more cells while having self-replication ability, and may be classified into totipotent stem cells, pluripotent stem cells, and multipotent stem cells. The stem cells may be appropriately selected without limitation depending on the purpose, and may be derived from adult cells, such as all known tissues, cells, and the like derived from mammals including humans, preferably humans, and for example, mesenchymal stem cells derived from fat, bone marrow, placenta (or placental tissue cells), or umbilical cord blood. In addition, the stem cells may refer to clonal stem cells.

The primed stem cells of the present invention may be stem cells enhanced with at least one selected from the group consisting of expression of TNFα-stimulated gene-6 (TSG6), expression of indoleamine 2,3-dioxygenase (IDO), expression of intercellular adhesion molecule 1 (ICAM1) and expression of vascular cell adhesion molecule (VCAM) compared with stem cells not treated with the combination of the priming factors. The primed stem cells may exhibit at least one atopic dermatitis therapeutic effect selected from the group consisting of reducing mast cells, reducing total IgE, increasing production of IgG2a, and reducing histamine.

When the stem cells of the present invention are used as a pharmaceutical composition, the pharmaceutical composition may further include suitable carriers, excipients and diluents commonly used in the preparation of the pharmaceutical composition. In addition, additives for solid or liquid formulations may be used in the preparation of the pharmaceutical composition. The additives for formulations may be either organic or inorganic.

Examples of the excipient may include lactose, sucrose, white sugar, glucose, corn starch, starch, talc, sorbitol, crystalline cellulose, dextrin, kaolin, calcium carbonate, silicon dioxide, and the like. Examples of the binder may include polyvinyl alcohol, polyvinyl ether, ethyl cellulose, methyl cellulose, arabic gum, tragacanth, gelatin, shellac, hydroxypropyl cellulose, hydroxypropyl methyl cellulose, calcium citrate, dextrin, pectin, and the like. Examples of the lubricant may include magnesium stearate, talc, polyethylene glycol, silica, hydrogenated vegetable oil, and the like. As a coloring agent, any coloring agent may be used as long as the coloring agent is permitted to be added to ordinary pharmaceuticals. These tablets and granules may be sugar-coated, gelatin-coated, or appropriately coated as needed. In addition, preservatives, antioxidants, etc. may be added as needed.

The pharmaceutical composition of the present invention may also be prepared in any formulation commonly prepared in the art (e.g., literature [Remington's Pharmaceutical Science, latest edition; Mack Publishing Company, Easton PA]), and the form of the formulation is not particularly limited, but preferably an external preparation. The external preparations of the present invention may include conventional external preparations such as sheets, liquid coating agents, sprays, lotions, creams, cataplasmas, powdered medicines, penetration pads, sprays, gels, pastas, liniments, ointments, aerosols, powders, suspensions, and transdermal absorbents. These formulations are described in the literature [Remington's Pharmaceutical Science], a generally known prescription in all pharmaceutical chemistry.

The pharmaceutically effective amount of the present invention may vary depending on a patient's wound type, an application site, a treatment frequency, treatment time, a dosage form, a patient's condition, a type of adjuvant, and the like. The used amount is not particularly limited, but may be 0.00001 to 10000 µg when the daily effective amount of the pharmaceutical composition of the present invention is applied to a patient. The daily dose may be administered once a day, divided into 2 to 3 times a day at appropriate intervals, or may be administered intermittently at intervals of several days.

However, since the used amount of the pharmaceutical composition of the present invention is determined according to many related factors such as a route of administration, patient's age, sex, weight, patient's severity, a type of wound, an application site, the number of treatments, treatment time, a dosage form, a patient's condition, a type of adjuvant, and the like, the effective amount should not be understood as limiting the scope of the present invention in any respect.

Further, the present invention provides a method for preventing or treating atopic dermatitis including treating stem cells treated with TNF-α, IFN-γ, and IFN-α; or TNF-α, IFN-γ, IFN-α, and vitamins to a subject in need thereof.

The subject of the present invention may mean any animal, including humans. These animals may be mammals such as cattle, horse, sheep, pig, goat, camel, antelope, dog, and cat that require treatment for similar symptoms to atopic dermatitis as well as humans. In addition, the animals may also refer to animals other than humans, but are not limited thereto.

The subject may include all patients in need of treatment for atopic dermatitis, including patients undergoing treatment for atopic dermatitis, patients who have previously been treated, and patients in need of treatment for atopic dermatitis.

In addition, the stem cells treated with TNF-α, IFN-γ and IFN-α; or TNF-α, IFN-γ, IFN-α, and vitamins of the present invention may be treated in combination with existing drugs or treatment methods for treating atopic dermatitis. When the primed stem cells of the present invention are treated in combination, the primed stem cells may be treated simultaneously or sequentially with other drugs or treatment methods for the treatment of atopic dermatitis.

Further, the present invention provides a stem cell therapeutic agent containing stem cells primed with TNF-α, IFN-γ and IFN-α; or TNF-α, IFN-γ, IFN-α, and vitamins for preventing or treating atopic dermatitis.

The stem cell therapeutic agent according to the present invention may be administered through various routes including oral, transdermal, subcutaneous, intravenous or intramuscular route, and the dose of the active ingredients may be appropriately selected according to several factors, such as a route of administration, the age, sex, and weight of a patient, and the severity of a patient. Preferably, the stem cell therapeutic agent may be administered parenterally, and may be administered by intravenous injection, subcutaneous injection, intramuscular injection, intraperitoneal injection, or the like.

The dose of the stem cell therapeutic agent of the present invention may be preferably 1 × 10² to 1 × 10¹² cells/kg per day.

Further, the present invention provides a cosmetic composition containing stem cells treated with TNF-α, IFN-γ, and IFN-α for preventing or alleviating atopic dermatitis.

More specifically, the TNF-α, IFN-γ, and IFN-α may be treated in a ratio of 0.1 to 3 : 0.1 to 3 : 0.1 to 3 (w/v), preferably 1 : 1 : 0.1 to 3 (w/v), more preferably 1 : 1 : 1 to 3 (w/v), and much more preferably 1 : 1 : 1 to 2.5 (w/v). In one embodiment of the present invention, the stem cells were treated with TNF-α, IFN-γ, and IFN-α at a concentration combination of 10 ng/ml, 10 ng/ml, and 20 ng/ml, and cultured to confirm an effect of preventing or alleviating atopic dermatitis of the stem cells.

In addition, the stem cells of the present invention may be stem cells further treated with vitamins in addition to the combination of TNF-α, IFN-γ, and IFN-α, and specifically, stem cells further treated with at least one vitamin selected from the group consisting of vitamin B2, vitamin B3, vitamin B5, and vitamin B6.

When the stem cells are primed by treatment with a combination of priming factors further containing vitamins, the reinforced function of the stem cells induced by treatment with TNF-α, IFN-γ and IFN-α may be more remarkably enhanced and the effect of preventing or alleviating atopic dermatitis may also be significantly improved. The TNF-α, IFN-γ, IFN-α and vitamins may be treated to the stem cells in a ratio of 0.1 to 3 : 0.1 to 3 : 0.1 to 3 : 100 to 10,000 (w/v), preferably 1 : 1 : 0.1 to 3 : 300 to 6,000 (w/v). More specifically, when vitamin B2 is added to TNF-α, IFN-γ, and IFN-α, the TNF-α, IFN-γ, IFN-α and vitamin may be treated in a ratio of 0.1 to 3 : 0.1 to 3 : 0.1 to 3 : 300 to 1,000 (w/v), more preferably 1 : 1 : 0.1 to 3 : 300 to 600 (w/v), much more preferably 1 : 1 : 0.1 to 3 : 400 to 550 (w/v), and even more preferably 1 : 1 : 2 : 500 (w/v). In addition, when vitamin B3, vitamin B5, or vitamin B6 is added to TNF-α, IFN-γ, and IFN-α, the TNF-α, IFN-γ, IFN-α and the vitamin may be treated in a ratio of 0.1 to 3 : 0.1 to 3 : 0.1 to 3 : 1,000 to 10,000 (w/v), more preferably 0.1 to 3 : 0.1 to 3 : 0.1 to 3 : 1,000 to 6,000 (w/v), much preferably 1 : 1 : 0.1 to 3 : 4,000 to 6,000 (w/v), even more specifically 1 : 1 : 0.1 to 3 : 4,000 to 5,500 (w/v), and even more preferably 1 : 1 : 2 : 5,000 (w/v).

The primed stem cells of the present invention may exhibit at least one effect of alleviating atopic dermatitis selected from the group consisting of reducing mast cells, reducing total IgE, increasing production of IgG2a, and reducing histamine.

Further, the present invention provides a method for preparing a composition for preventing or treating atopic dermatitis including culturing stem cells treated with TNF-α, IFN-γ and IFN-α; or TNF-α, IFN-γ, IFN-α, and vitamins. The vitamins may be at least one vitamin selected from the group consisting of vitamin B2, vitamin B3, vitamin B5, and vitamin B6.

According to the present invention, when the stem cells are treated with TNF-α, IFN-γ and IFN-α; or TNF-α, IFN-γ, IFN-α, and vitamins and cultured, stem cells with reinforced functions for preventing, alleviating, or treating atopic dermatitis may be prepared. Particularly, in one embodiment of the present invention, it was confirmed that stem cells with an enhanced treatment effect on atopic dermatitis may be produced by treatment with priming factors of a combination of TNF-α, IFN-γ, IFN-α, and vitamin B6, and these stem cells were called `primed clonal mesenchymal stem cell 2 (pcMSC2)'.

Further, the present invention provides a use of TNF-α, IFN-γ and IFN-α; or TNF-α, IFN-γ, IFN-α, and vitamins to be used for a method for preparing a composition for preventing or treating atopic dermatitis including 1) culturing stem cells treated with TNF-α, IFN-γ and IFN-α; or TNF-α, IFN-γ, IFN-α, and vitamins. The vitamins may be at least one vitamin selected from the group consisting of vitamin B2, vitamin B3, vitamin B5, and vitamin B6, more preferably vitamin B6.

The treatment may mean treating stem cells with a combination of TNF-α, IFN-γ, and IFN-α, or a combination of further containing at least one vitamin selected from the group consisting of vitamin B2, vitamin B3, vitamin B5, and vitamin B6 in the combination for 12 hours to 36 hours, preferably 20 hours to 25 hours, and then culturing the stem cells. For the culture, stem cell culture media widely known in the art may be used without limitation.

### [Modes for the Invention]

Hereinafter, the present invention will be described in more detail through Examples. These Examples are just illustrative of the present invention, and it will be apparent to those skilled in the art that it is not interpreted that the scope of the present invention is limited to these Examples.

### Example 1. Culture of stem cells and selection of candidate substances

In a 37°C and 5% CO₂ incubator, mesenchymal stem cells in a storage state (LN₂ tank storage) were thawed and cultured, and at this time, the cells were cultured in a medium (DMEM, alpha-MEM) containing 10% FBS or 4% hPL until proliferated to a cell confluence of about 80%. The cultured mesenchymal stem cells were seeded in a 100 mm dish, and treated with candidate substances for reinforcing the functions of the mesenchymal stem cells for 24 hours, and then the concentrations of primary candidate substances for reinforcing the functions were set.

TNF-α and IFN-γIPN-α were selected as the primary candidate substances, and in order to confirm the lowest effective concentration capable of increasing the expression of TNFα-stimulated gene-6 (TSG6) and indoleamine 2,3-dioxygenase (IDO) to confirm the reinforced functions, the stem cells were treated with 5, 10, and 20 ng/ml of TNF-α, 5, 10, and 20 ng/ml of IFN-γ, and 10, 20, and 40 ng/ml of IFN-α, respectively, and changes in expression of TSG6 and IDO were confirmed.

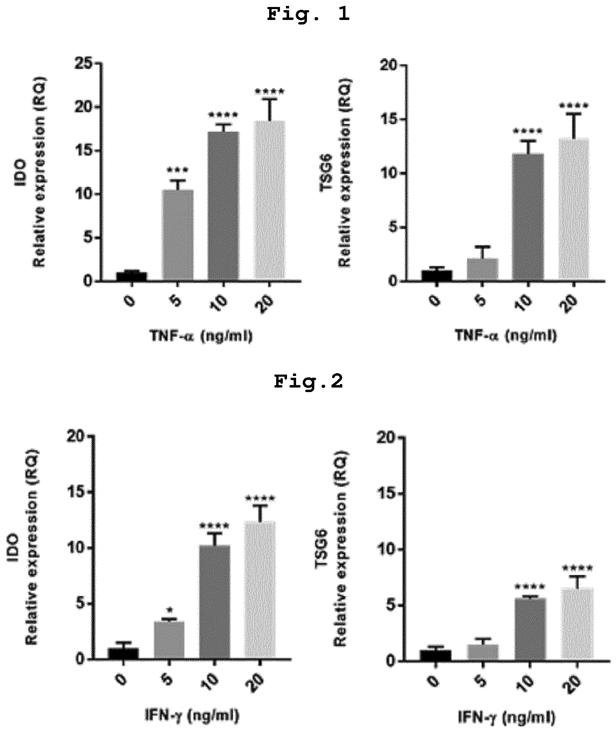

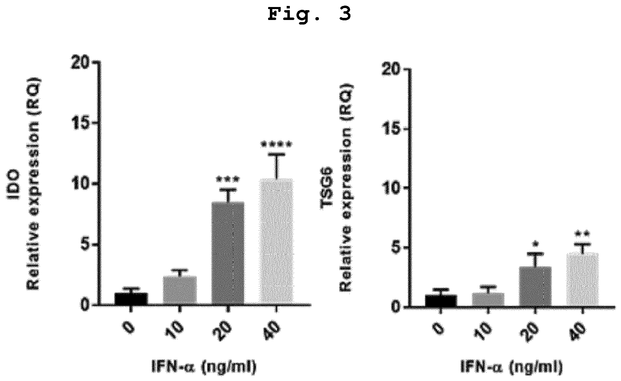

The IDO was known as an immunomodulatory factor that inhibited the proliferation of immune cells such as T cells by converting tryptophan, which was essential for T cell proliferation, into kynurenine, and the TSG6 was known as an anti-inflammatory regulator secreted by mesenchymal stem cells. After culturing the stem cells, total RNA was isolated using TRIzol (Invitrogen), and cDNA was synthesized from total RNA using PrimeScript^{™} RT reagent Kit with gDNA Eraser (TaKaRa), and qRT-PCR was performed. The results of TSG6 and IDO according to the concentration of each candidate substance were illustrated in FIGS. 1, 2 and 3.

As illustrated in FIGS. 1 to 3, 10 ng/ml of TNF-α, 10 ng/ml of IFN-γ, and 20 ng/ml of IFN-α were confirmed as the lowest effective concentrations capable of inducing significantly increased expression of both TSG6 and IDO, and thereafter, the experiment was performed based on the corresponding concentrations.

### Example 2. Selection of combination for reinforcing functions of stem cells

Through Example 1, candidate substances and concentration combinations thereof selected to search for more functionally reinforced compositions based on the candidate substances and the lowest effective concentrations confirmed to induce the reinforced functions of stem cells were set as shown in Table 1. Mesenchymal stem cells were treated with the candidate combinations described in Table 1 below for 24 hours, and the stem cells were cultured as in Example 1, and then total RNA was isolated using TRIzol (Invitrogen). Thereafter, qRT-PCR was performed by synthesizing cDNA from total RNA using the PrimeScript^{™} RT reagent Kit with gDNA Eraser (TaKaRa).

**[Table 1]**

| **Experimental Group** | |
|---|---|
| 0 | **Control (untreated group)** |
| 1 | TNF-α (10ng/ml) |
| 2 | IFN-γ (10ng/ml) |
| 3 | IFN-α (20ng/ml) |
| 4 | TNF-α (10ng/ml) + IFN-γ (10ng/ml) |
| 5 | TNF-α (10ng/ml) + IFN-α (20ng/ml) |
| 6 | IPN-γ (10ng/ml) + IFN-α (20ng/ml) |
| 7 | TNF-α (10ng/ml) + IFN-γ (10ng/ml) + IFN-α (20ng/ml) |

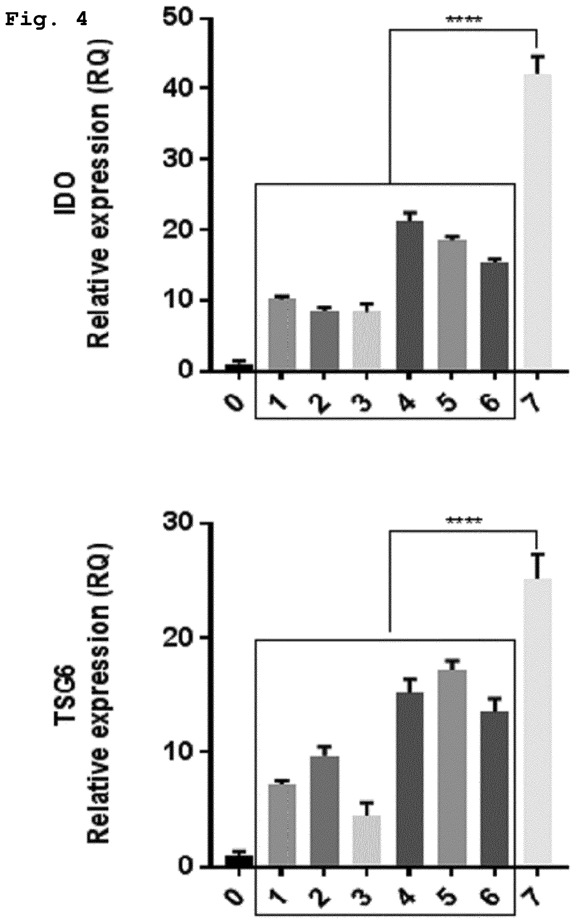

Through this, changes in the expression of IDO and TSG6, which were representative factors of immune and anti-inflammatory regulation, were confirmed, and the results were shown in FIG. 4.

As illustrated in FIG. 4, compared to an untreated control group 0, increased expression of IDO and TSG6 was confirmed in treatment groups of all single candidate substances, TNF-α, IFN-γ, and IFN-α (Experimental Groups 1 to 3), but in Experimental Groups 4 to 6 in which these substances were combined, a more significant increase in expression of IDO and TSG6 was confirmed compared to candidate substance-alone treated groups. In particular, Experimental Groups 4 and 5, in which TNF-α was treated with IFN-γ or IFN-α, excellent effects were exhibited compared to Experimental Group 6, in which only IFN-γ and IFN-α were combined, and surprisingly, in a treatment group in which all three candidate substances were combined, a function reinforcing effect which was twice or more increased than that of a combination of two substances was confirmed.

Therefore, through the results, when the stem cells were treated with a combination of TNF-α (10 ng/ml) + IFN-γ (10 ng/ml) + IFN-α (20 ng/ml), it was confirmed that very significantly reinforced immunity and anti-inflammatory functions of the stem cells could be achieved.

### Example 3. Confirmation of function reinforcing effect of stem cells by adding vitamins

### 3.1 Confirmation of function reinforcing effect of stem cells by adding vitamin alone

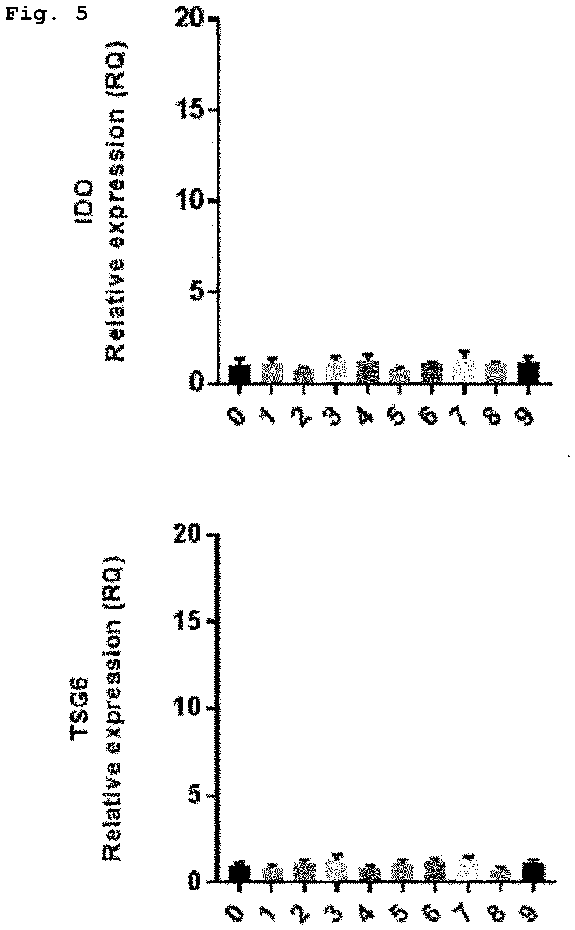

Vitamins were known to improve the proliferative ability of stem cells and maintain the sternness of stem cells when added during a stem cell culture process. Accordingly, various vitamins were treated in the stem cell culture process, and experiments were performed to confirm whether or not vitamins could achieve anti-inflammatory and immune function reinforcing effects of stem cells. Specifically, the stem cells were treated with various types of vitamins shown in Table 2 according to the method described in Example 1, and the results of confirming changes in expression of IDO and TSG6 according to treatment with each candidate substance were illustrated in FIG. 5.

**[Table 2]**

| **Experimental Group** | |
|---|---|
| **0** | **Control (untreated group)** |
| **1** | **Vitamin A 10ug/ml** |
| **2** | **Vitamin B1 50ug/ml** |
| **3** | **Vitamin B2 5ug/ml** |
| **4** | **Vitamin B3 50ug/ml** |
| **5** | **Vitamin B5 50ug/ml** |
| **6** | **Vitamin B6 50ug/ml** |
| **7** | **Vitamin B12 50ug/ml** |
| **8** | **Vitamin D2 10ug/ml** |
| **9** | **Vitamin D3 10ug/ml** |

As illustrated in FIG. 5, the vitamin-treated groups did not show significant effects on the changes in expression of IDO and TSG6 involved in the anti-inflammatory and immunomodulatory functions of the stem cells.

### 3.2 Confirmation of stem cell function reinforcing effect according to combination of vitamin and TNF-α + IFN-γ + IFN-α

As described above, when vitamins, which did not show an effect of reinforcing the anti-inflammatory and immunomodulatory functions of the stem cells as a single substance, were added to a combination of functionally reinforced substances identified in Example 2, in order to confirm whether or not to exhibit a synergistic effect, as shown in Table 3 below, each vitamin was added to TNF-α (10 ng/ml) + IFN-γ (10 ng/ml) + IFN-α (20 ng/ml), changes in expression of IDO and TSG6 according to each combination treatment were confirmed, and the results were shown in FIG. 6.

**[Table 3]**

| **Experimental Group** | |
|---|---|
| 0 | **Control (untreated group)** |
| 1 | TNF-α (10ng/ml) + IFN-γ (10ng/ml) + IFN-α (20ng/ml) |
| 2 | TNF-α (10ng/ml) + IFN-γ (10ng/ml) + IFN-α (20ng/ml) + vitamin A 10ug/ml |
| 3 | TNF-α (10ng/ml) + IFN-γ (10ng/ml) + IFN-α (20ng/ml) + vitamin B1 50ug/ml |
| 4 | TNF-α (10ng/ml) + IFN-γ (10ng/ml) + IFN-α (20ng/ml) + vitamin B2 5ug/ml |
| 5 | TNF-α (10ng/ml) + IFN-γ (10ng/ml) + IFN-α (20ng/ml) + vitamin B3 50ug/ml |
| 6 | TNF-α (10ng/ml) + IFN-γ (10ng/ml) + IFN-α (20ng/ml) + vitamin B5 50ug/ml |
| 7 | TNF-α (10ng/ml) + IFN-γ (10ng/ml) + IFN-α (20ng/ml) + vitamin B6 50ug/ml |
| 8 | TNF-α (10ng/ml) + IFN-γ (10ng/ml) + IFN-α (20ng/ml) + vitamin B12 50ug/ml |
| 9 | TNF-α (10ng/ml) + IFN-γ (10ng/ml) + IFN-α (20ng/ml) + vitamin D2 10ug/ml |
| 10 | TNF-α (10ng/ml) + IFN-γ (10ng/ml) + IFN-α (20ng/ml) + vitamin D3 10ug/ml |

As shown in FIG. 6, the expression of IDO and TSG6 was not significantly increased in Experimental Groups 2, 3, 8, and 9 compared to Experimental Group 1 without vitamin treatment, and in Experimental Group 10, the expression of TSG6 was increased, but the increase in expression of IDO was not confirmed compared to the control group. On the other hand, in the case of vitamin B2 (Experimental Group 4), vitamin B3 (Experimental Group 5), vitamin B5 (Experimental Group 6), and vitamin B6 (Experimental Group 7), it was confirmed that the expression of both IDO and TSG6 was increased significantly. It was confirmed that in the case of TSG6, as compared to Experimental Group 1 treated with TNF-α + IFN-γ + IFN-α, in Experimental Groups 4, 5, 6, and 7, the expression was increased by 49%, 30%, 35%, and 45%, respectively, and in the case of IDO, as compared to Experimental Group 1, in Experimental Groups 4, 5, 6, and 7, the expression was increased by 25%, 37%, 31%, and 18%, respectively.

As a result, in the case of treating stem cells with vitamins alone, the anti-inflammatory and immunomodulatory ability of the stem cells were not improved, but in the case of treating a combination of TNF-α + IFN-γ + IFN-α and vitamin B2, vitamin B3, vitamin B5 or vitamin B6, the effect of TNF-α + IFN-γ + IFN-α may be further enhanced.

### Example 4. Confirmation of changes in expression of ICAM1 and VCAM

ICAM1 and VCAM may exhibit effects on various immune and inflammatory-related diseases by inhibiting proliferation and inducing death of T cells through T cell binding to reduce excessive immune and inflammatory responses. Accordingly, mesenchymal stem cells were treated for 24 hours with the same Experimental Group described in Table 3 of Example 3 above, and the stem cells were cultured as in Example 1, and then total RNA was isolated using TRIzol (Invitrogen). Then, qRT-PCR was performed by synthesizing cDNA from total RNA using the PrimeScript^{™} RT reagent Kit with gDNA Eraser (TaKaRa).

In order to confirm the enhancement of the effect of stem cells for treating immune diseases and inflammatory diseases, the expression of intercellular adhesion molecule 1 (ICAM1) and vascular cell adhesion molecule (VCAM), which were adherent factors expressed on the surface of stem cells, were confirmed, and the results were illustrated in FIG. 7.

As illustrated in FIG. 7, it was confirmed that in the case of ICAM1, compared to Experimental Group 1 treated with TNF-α + IFN-γ + IFN-α, in Experimental Groups 4, 5, 6, and 7, the expression was increased by 45%, 100%, 35%, and 45%, respectively, and in the case of VACM, compared to Experimental Group 1, in Experimental Groups 4, 5, 6, and 7, the expression was increased by 52%, 69%, 52%, and 69%, respectively.

Therefore, when the stem cells were treated with a combination of TNF-α + IFN-γIFN-α and vitamin B2, vitamin B3, vitamin B5 or vitamin B6, the expression of ICAM1 and VCAM was significantly increased, and as a result, the immunomodulatory activity and the anti-inflammatory effect of the stem cells may be promoted.

### Example 5. Confirmation of inflammatory and immunomodulatory ability

It was confirmed whether the combination of priming conditions identified in Example 4, which enhanced immune and inflammatory regulators such as IDO, TSG6, ICAM-1, and VCAM, actually inhibited inflammatory and immune responses under conditions for inducing excessive immune responses, and exhibited a superior effect to cMSCs whose functions were not reinforced. Hereinafter, cMSCs were treated with the combination of TNF-α + IFN-γIFN-α and vitamin B6, Experimental Group 7 in Table 3, and MSC primed with the combination was named `primed clonal mesenchymal stem cell 2 (pcMSC2)'.

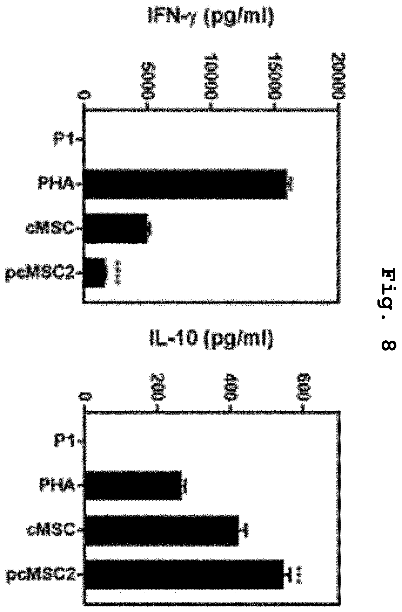

A phytochemagglutinin (PHA)-stimulated lymphocyte culture experiment was a method for inducing excessive immune activity by a method of using a reagent PHA for inducing cell division stimulation of lymphocytes. Peripheral blood mononuclear cells (PBMCs, peripheral blood cells) were dispensed at 2 × 10⁵ cells per well in a 96-well plate, stimulated with 1 µg/ml of PHA, and co-cultured with 5 × 10⁴ cells of cMSC or pcMSC2 for 4 days. After the culture was completed, the supernatant was collected and inflammatory cytokine, IFN-gamma, and anti-inflammatory cytokine, IL-10, were confirmed through ELISA, and the results were illustrated in FIG. 8. As a negative control group, a group not stimulated with PHA was used, and was denoted as P1.

As illustrated in FIG. 8, it was confirmed that when stimulated with PHA, IFN-gamma increased significantly to 16,013 pg/ml, but when co-cultured with cMSC, IFN-gamma decreased by 5,048 pg/ml, and when co-cultured with pcMSC2, IFN-gamma significantly increased to 1,700 pg/ml. In addition, when comparing cMSC and pcMSC2, it was confirmed that the function-reinforced pcMSC2 showed a significant decrease in IFN-gamma by about 66% compared to cMSC. In the case of the anti-inflammatory cytokine IL-10, an increase in IL-10 was confirmed in both cMSC and pcMSC2, and in particular, under co-culture conditions with pcMSC2, which was function-reinforced compared to cMSC, about 22% more IL-10 was secreted.

### Example 6. Confirmation of therapeutic effect of pcMSC2 in atopic dermatitis animal model

An experiment was performed to confirm whether even in atopic dermatitis animal models, pcMSC2 of the present invention exhibited superior immunomodulatory and anti-inflammatory effects to unprimed cMSCs.

### 6.1 construction and experimental method of atopic dermatitis animal model

Animals used in an atopic dermatitis test were female 6-week-old SPF (specific pathogen-free) BALB/c mice (15 to 20 g) and supplied by SLC, Inc (Shizuoka, Japan) and kept in an acclimatization period in the animal room for 1 week, and then the experiment was conducted. At the Life Science Research Institute of Inha University College of Medicine, five mice were bred in a mouse cage at a temperature of 22 to 25°C, humidity of 40 to 60%, and in lighting of 150 to 300 Lux in 12-hour shifts day and night, and a breeding environment was maintained to freely consume sterilized distilled water and solid feed and the experiment was performed. 7-week-old female BALB/c mice were administered with a mixture of Ovalbumin (OVA) 50 µg/50 µl (PBS) and Alum Adjuvant 4 mg/100 µl intraperitoneally and subcutaneously once a week for a total of 3 times for 3 weeks to induce the sensitization of atopic dermatitis. Since the mixture was administered to the same area all three times to cause inflammation unrelated to the corresponding disease, the mixture was administered to different areas, respectively. After three weeks of OVA administration, a 1.2 × 1.2 cm-sized sterile gauze soaked in OVA 60 µg/60 µl (PBS) was attached to the shaved back skin 2 to 3 times a week for 2 weeks to induce localized atopy. After inducing atopy through an OVA patch, PBS, cMSC, and pMSC2 were injected through the caudal vein a total of 3 times at 2-day intervals. The injection volume was administered three times by 1.66 × 10⁵/200 µl each time, for a total of 5.0 × 10⁵/600 µl intravenously. After two days of completing the administration, the lesion area was shaved again, and then a 1.2 × 1.2 cm-sized sterile gauze soaked in OVA 60 µg/60 µl (PBS) was attached to the shaved back skin 3 to 4 times a week for 2 weeks to reinduce localized atopy. At 13 to 14 days after the induction of atopic dermatitis, the experimental animals were corrected in a restrainer and administered via the caudal vein using a 1 ml syringe from BD equipped with a 26 1/2 gauge needle. Animals in a Veh. group were intravenously administered with PBS instead of a test material in the same manner, and except for Naive and Veh. groups, the test material was suspended in PBS and administered intravenously. Both PBS and the test material were administered in the same amount of 200 µl/head.

To confirm the effect on atopic dermatitis, blood samples were obtained in the following manner. All experimental animals were anesthetized with isoflurane on day 13 after administration of a cell stabilizer or test material, and then the abdomen was incised to expose the posterior vena cava, and about 700 µl of blood was collected using a 1 ml syringe. The collected blood was contained in a serum separate tube and centrifuged at 3,000 rpm/15 minutes at 4°C to separate the serum, and the separated serum was contained in a 1.5 ml e-tube and stored in a -70°C deep freezer.

To analyze the effect on atopic dermatitis, the concentrations of Total IgE, IgG2a, and histamine were measured in serum samples of all experimental animals using an ELISA kit, and the measurement method was performed according to the protocol in the kit. Specifically, the measurement of the IgE level in the blood was performed by first containing blood collected from the posterior vena cava in an EDTA tube, centrifuging the blood at 3,000 rpm/15 minutes, separating only the supernatant, and storing the supernatant at -70°C until the experiment. A capture antibody capable of recognizing IgE was stored in the ELISA plate with a coating buffer at 4°C overnight, and the next day, after a blocking process for 1 hour, the serum was diluted 100 times and reacted in an amount of 50 µl at room temperature for 2 hours. Thereafter, a secondary antibody recognizing IgE was added, added with a TMB solution as a substrate buffer, reacted for about 15 minutes, and then added with 50 µl of a stop solution. The values were measured at a filter of 450 nm using an ELISA reader.

At the end of the experiment, the extracted skin tissue was immobilized in a 4% formalin solution, sectioned, and then mast cells infiltrated in the dermis were stained with toluidine blue.

### 6.2 Confirmation of alleviation of skin lesions by pcMSC2

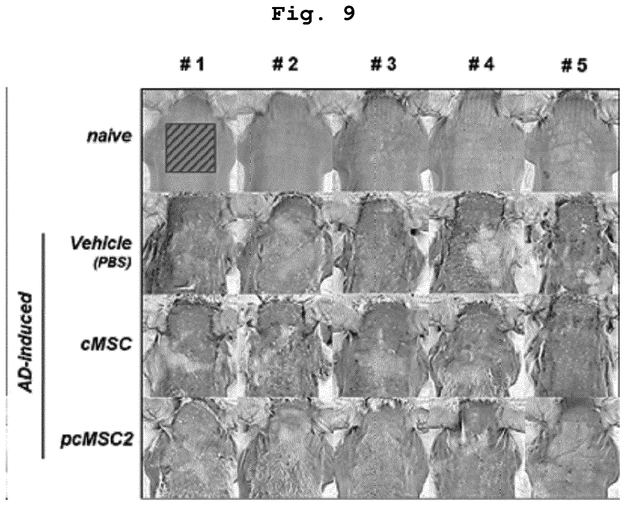

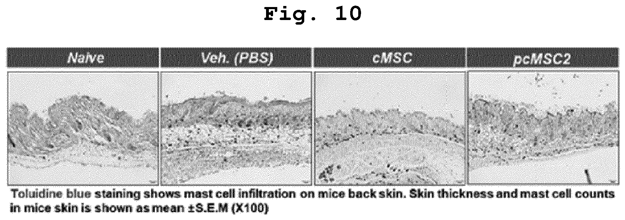

cMSCs and pcMSC2 were administered to the atopic dermatitis-induced animal model of Example 6.1 using the same protocol, and changes in skin lesions were confirmed after 59 days, and the results were illustrated in FIGS. 9 and 10.

As illustrated in FIG. 9, it was confirmed that atopic skin lesions in the pcMSC2-administered group were effectively alleviated even with the naked eye. In particular, as illustrated in FIG. 10, it was confirmed that pachyderma was more effectively alleviated in the pcMSC2-administered group than a non-primed cMSC-administered group.

### 6.3. Confirmation of mast cell reduction effect of pcMSC2

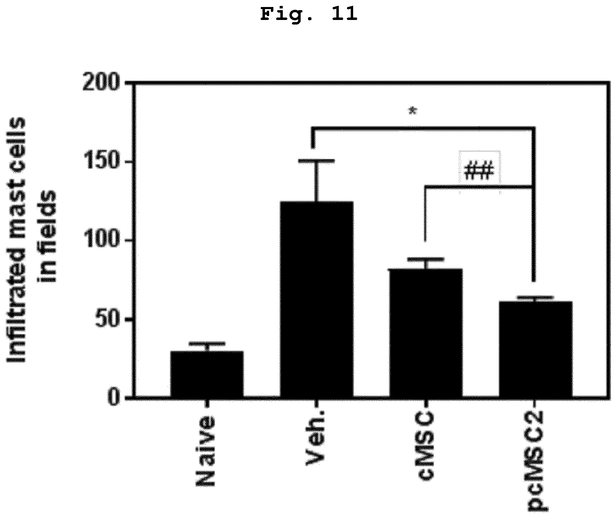

cMSCs and pcMSC2 were administered to the atopic dermatitis-induced animal model in Example 6.1 using the same protocol and skin tissue staining was additionally performed using Giemsa, and then the number of infiltrated mast cells was counted using paint.NET at 100x magnification. The number of mast cells counted was quantified and illustrated in FIG. 11.

As illustrated in FIG. 11, it was confirmed that compared to a normal control group, in the atopic dermatitis model, mast cells were significantly increased by inflammation induction, and such an increase was reduced by cMSC and pcMSC2. In particular, when comparing cMSC and pcMSC2, it was confirmed that the number of mast cells was significantly reduced in the pcMSC2-treated group.

### 6.4. Confirmation of total IgE inhibition effect and IgG2 production effect of pcMSC2

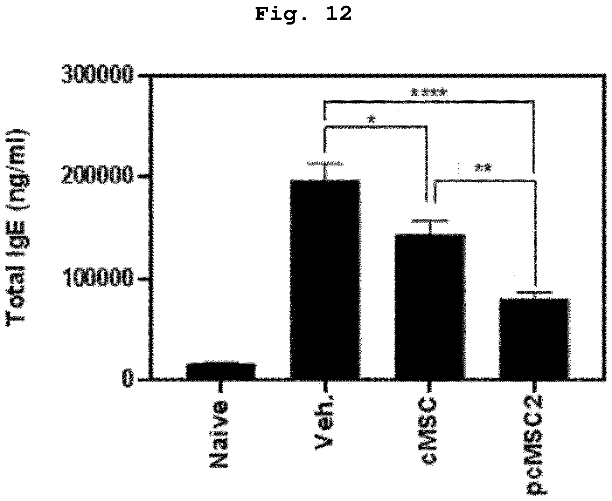

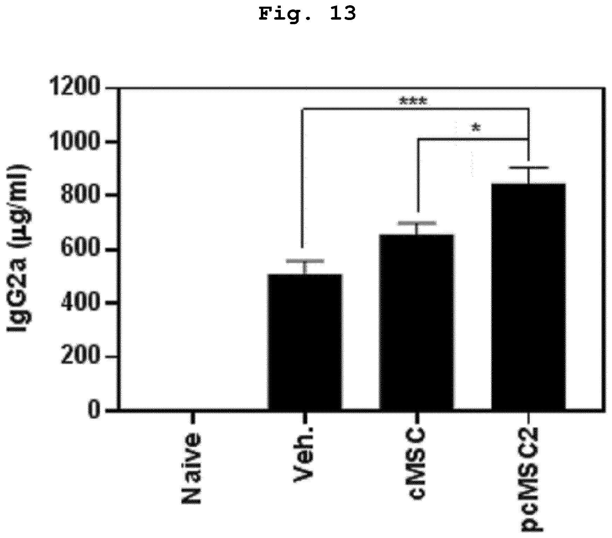

cMSCs and pcMSC2 were administered to the atopic dermatitis-induced animal model of Example 6.1 using the same protocol, and total IgE and IgG2a were compared, and the results were illustrated in FIGS. 12 and 13.

As illustrated in FIG. 12, it was confirmed that total IgE, which was known to exist at a high concentration in the serum of atopic patients, decreased in the cMSC and pcMSC2-administered groups, and particularly, significantly decreased in the pcMSC2-administered group compared to cMSC. In addition, as illustrated in FIG. 13, it was confirmed that the production of IgG2a capable of alleviating atopic symptoms by controlling Th1 shifting and immune balance was also significantly better in pcMSC2 than in cMSC, and approximately twice increased compared to the atopic dermatitis model group (vehicle).

### 6.5. Confirmation of histamine reduction effect of pcMSC2

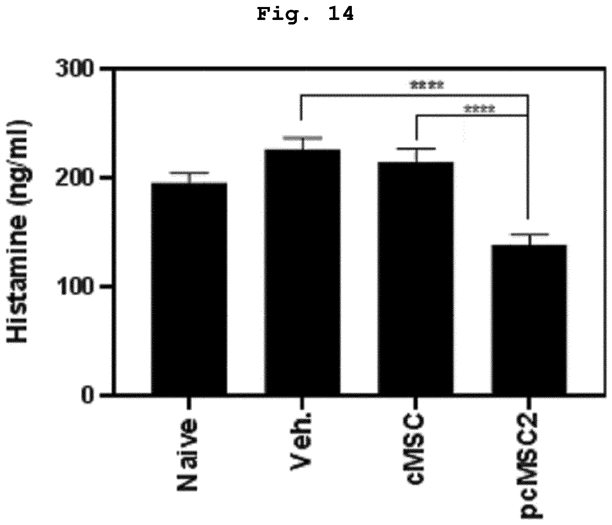

Histamine was known to cause itching by dilating blood vessels and stimulating nerve fibers sensing itchiness. cMSCs and pcMSC2 were administered to the atopic dermatitis-induced animal model of Example 6.1 using the same protocol, and histamines were compared, and the results were illustrated in FIG. 14.

As illustrated in FIG. 14, there was no significant change in histamine in the cMSC-treated group compared to the vehicle, but a significant reduction effect of histamine was confirmed in the primed pcMSC2-administered group.

Through the above contents, it was confirmed that when the stem cells were treated with TNF-α, IFN-γ and IFN-α; or a combination of TNF-α + IFN-γ + IFN-α and vitamin B2, vitamin B3, vitamin B5 or vitamin B6, the stem cells were primed to improve the immunomodulatory and inflammatory regulation ability of stem cells. Particularly, it was confirmed that the stem cells treated with the primed composition containing the combination of TNF-α, IFN-γ, IFN-α and vitamin B6 had a superior atopic dermatitis therapeutic effect to non-primed stem cells.

As described above, specific parts of the present invention have been described in detail, and it will be apparent to those skilled in the art that these specific techniques are merely preferred embodiments, and the scope of the present invention is not limited thereto. Therefore, the substantial scope of the present invention will be defined by the appended claims and their equivalents.

## Claims

1. A pharmaceutical composition for preventing or treating atopic dermatitis comprising stem cells treated with TNF-α, IFN-γ, and IFN-α.

2. The pharmaceutical composition for preventing or treating atopic dermatitis of claim 1, wherein the TNF-α, IFN-γ and IFN-α are treated in a ratio of 0.1 to 3 : 0.1 to 3 : 0.1 to 3 (w/v).

3. The pharmaceutical composition for preventing or treating atopic dermatitis of claim 1, wherein the stem cells are stem cells further treated with at least one vitamin selected from the group consisting of vitamin B2, vitamin B3, vitamin B5 and vitamin B6.

4. The pharmaceutical composition for preventing or treating atopic dermatitis of claim 3, wherein the TNF-α, IFN-γ, IFN-α and the vitamin are treated in a ratio of 0.1 to 3 : 0.1 to 3 : 0.1 to 3 : 100 to 10,000 (w/v).

5. The pharmaceutical composition for preventing or treating atopic dermatitis of any one of claims 1 to 4, wherein the stem cells are mesenchymal stem cells derived from fat, bone marrow, placenta, or umbilical cord blood.

6. The pharmaceutical composition for preventing or treating atopic dermatitis of any one of claims 1 to 4, wherein the stem cells are improved with at least one selected from the group consisting of expression of TNFα-stimulated gene-6 (TSG6), expression of indoleamine 2,3-dioxygenase (IDO), expression of intercellular adhesion molecule 1 (ICAM1) and expression of vascular cell adhesion molecule (VCAM).

7. The pharmaceutical composition for preventing or treating atopic dermatitis of any one of claims 1 to 4, wherein the stem cells exhibit at least one atopic dermatitis therapeutic effect selected from the group consisting of reducing mast cells, reducing total IgE, increasing production of IgG2a, and reducing histamine.

8. A stem cell therapeutic agent for preventing or treating atopic dermatitis, comprising the composition of any one of claims 1 to 4.

9. A cosmetic composition for preventing or alleviating atopic dermatitis comprising stem cells treated with TNF-α, IFN-γ, and IFN-α.

10. The cosmetic composition for preventing or alleviating atopic dermatitis of claim 9, wherein the TNF-α, IFN-γ and IFN-α are treated in a ratio of 0.1 to 3 : 0.1 to 3 : 0.1 to 3 (w/v).

11. The cosmetic composition for preventing or alleviating atopic dermatitis of claim 9, wherein the stem cells are stem cells further treated with at least one vitamin selected from the group consisting of vitamin B2, vitamin B3, vitamin B5 and vitamin B6.

12. The cosmetic composition for preventing or alleviating atopic dermatitis of claim 9, wherein the TNF-α, IFN-γ, IFN-α and the vitamin are treated in a ratio of 0.1 to 3 : 0.1 to 3 : 0.1 to 3 : 100 to 10,000 (w/v).

13. The cosmetic composition for preventing or alleviating atopic dermatitis of any one of claims 9 to 12, wherein the stem cells exhibit at least one atopic dermatitis alleviating effect selected from the group consisting of reducing mast cells, reducing total IgE, increasing production of IgG2a, and reducing histamine.

14. A method for preparing a composition for preventing or treating atopic dermatitis, comprising 1) culturing stem cells treated with TNF-α, IFN-γ, and IFN-α.

15. The method for preparing the composition for preventing or treating atopic dermatitis of claim 14, wherein in step 1), the stem cells are further treated with at least one vitamin selected from the group consisting of vitamin B2, vitamin B3, vitamin B5 and vitamin B6.

16. A method for preventing or treating atopic dermatitis comprising treating stem cells primed by TNF-α, IFN-γ, and IFN-α; or TNF-α, IFN-γ, IFN-α, and vitamins to a subject in need thereof.
